# EUROPEAN PATENT APPLICATION

(11) **EP 4 635 398 A2**
(43) Date of publication of application: **22.10.2025**
(21) Application number: 25186187.8
(22) Date of filing: 24.09.2015
(51) Int. Cl.: A61B 1/012

(54) **COATED BALLOONS AND COATED BALLOON ASSEMBLIES AND RELATED METHODS OF USE AND MANUFACTURE**

(30) Priority: 25.09.2014 US 201462055384 P
(62) Divisional of application: 15844212.9
(71) Applicant: Merit Medical Systems, Inc., South Jordan UT 84095 (US)
(72) Inventor: HOPKINSON, Aaron J, Herriman, UT 84096 (US); WIERSDORF, Jason Matthew, West Jordan, UT 84081 (US)
(74) Representative: Cleveland Scott York

(57) **Abstract**

Medical devices and medical assemblies that have a lubricant disposed on an inner surface (e.g., balloons to be inflated within a cavity of a patient) are disclosed, along with related methods of use and manufacture. Such devices or assemblies may be passed through an elongate channel (e.g., a catheter channel or the working channel of an endoscope) and deployed distal of the elongate channel to adopt an expanded configuration. The devices or assemblies may then be compacted as they are withdrawn into the elongate channel after deployment. Some medical devices and assemblies comprising a lubricant on an inner surface of a balloon body may be more readily withdrawn into the elongate channel than embodiments that lack such a lubricant.

## Description

### CROSS-REFERENCE TO RELATED APPLICATIONS

This patent application claims priority to U.S. Provisional Application No. 62/055,384 entitled COATED BALLOONS AND COATED BALLOON ASSEMBLIES AND RELATED METHODS OF USE AND MANUFACTURE, filed on September 25, 2014, which is incorporated herein by reference in its entirety.

### TECHNICAL FIELD

This application generally relates to medical devices and assemblies for use in medical procedures, along with related methods. In some embodiments, a medical device may comprise a balloon or other device that is configured to adopt an expanded configuration after having been passed through an elongate channel. The medical device may comprise a lubricious inner coating that facilitates compaction of the medical device when the device is displaced within the elongate channel.

### BRIEF DESCRIPTION OF THE DRAWINGS

The written disclosure herein describes illustrative embodiments that are non-limiting and non-exhaustive. Reference is made to certain of such illustrative embodiments that are depicted in the figures, in which:
FIG. 1 is a front view of a medical assembly with a balloon in a first compact configuration and disposed distal of a channel of an elongate medical device.
FIG. 2 is a front view of the medical assembly of FIG. 1 with the balloon in an inflated and expanded configuration.
FIG. 3 is a front view of the medical assembly of FIG. 1 with the balloon in a second compact configuration.
FIG. 4 is a front view of the medical assembly of FIG. 1 depicting withdrawal of the balloon into the channel of the elongate medical device after the balloon has been deflated.
FIG. 5 is a cut-away perspective view of a balloon, according to another embodiment, in an inflated configuration.
FIG. 6 is a cross-sectional front view of a balloon body, according to another embodiment, that is being filled with a solution for providing a lubricant layer on an inner surface of the balloon body.
FIG. 7 is a cross-sectional front view of the balloon body of FIG. 6 with a lubricant layer on the inner surface of the balloon body and air being forced through the interior of the balloon body.
FIG. 8 is a column scatter graph depicting the maximum force needed to withdraw various balloon bodies into a particular working channel of an endoscope, where the balloon bodies differ with regard to whether and what type of lubricant layer is disposed on their inner surfaces.
FIG. 9 is a cross-sectional view of an inverted balloon segment where a lubricant layer is disposed on an inner surface of a balloon segment and the inverted balloon is disposed between balloon material that is attached to silicone pads.
FIG. 10 is a cross-sectional view of an inverted balloon segment that lacks lubrication where the balloon segment is disposed between balloon material that is attached to silicone pads.
FIG. 11 is a column scatter graph depicting the test peak friction force of various balloon segments.

### DETAILED DESCRIPTION

It will be readily understood by one of skill in the art having the benefit of this disclosure that the components of the embodiments as generally described and illustrated in the figures herein could be arranged and designed in a wide variety of different configurations. Thus, the following more detailed description of various embodiments, as represented in the figures, is not intended to limit the scope of the present disclosure, but is merely representative of various embodiments. While various aspects of the embodiments are presented in drawings, the drawings are not necessarily drawn to scale unless specifically indicated.

The phrase "coupled to" is used in its ordinary sense, and is broad enough to refer to any suitable coupling or other form of interaction between two or more entities, including mechanical, fluid, and thermal interaction. Two components may be coupled to each other even though they are not in direct contact with each other. For example, two components may be coupled to each other through an intermediate component. The phrase "fluid communication" is used in its ordinary sense, and is broad enough to refer to arrangements in which a fluid (e.g., a gas or a liquid) can flow from one element to another element when the elements are in fluid communication with each other.

The directional terms "proximal" and "distal" are generally used in their ordinary sense. More particularly, when used in connection with a device or component, these terms generally refer to opposite locations on the device or component. The proximal end of a component or device is the end of the device closest to the practitioner when the device is in normal use. The distal end is opposite the proximal end, along the longitudinal axis of the device, or the end farthest from the practitioner during normal use. For example, with regard to elongate devices that are typically disposed within a patient, the proximal end may be the end of the device that sticks out of the patient, while the distal end of the device is the end that is initially inserted into the patient.

An "inner surface" of a balloon or balloon segment refers to the surface on the interior of the balloon when the balloon is in normal operation (or the corresponding surface of a balloon segment). For example, the exposed external surface of an inverted balloon or balloon segment is an inner surface.

The terms "lubricant," "lubricious," etc. are to be given their ordinary meaning as would be understood by one of ordinary skill in the art with the benefit of this disclosure. The term "lubricant layer," as used herein, is specifically defined as a layer comprising a substance (e.g., a solid, liquid, or gel), other than an aqueous solution, that causes a reduction in friction between adjacent surfaces. Although a lubricant layer cannot consist of an aqueous solution, the term "lubricant layer" encompasses, *inter alia,* a layer of a non-aqueous substance that (1) is disposed on an inner surface of a balloon body and (2) decreases friction between adjacent inner surfaces of the balloon body by retaining inflation liquid (e.g., an aqueous liquid) to a greater extent than would the inner surface of the balloon body in the absence of the lubricant layer. For example, a hydrophilic polymer layer that decreases friction by attracting water from an aqueous solution is within the scope of this definition.

The "test peak friction force" for a particular balloon segment is the force measured as set forth in Example 3. An "acute angle," as used herein, is an angle that is 90° or less. Stated differently, as used herein, an acute angle includes a right angle.

In some medical procedures, a balloon or other medical device (e.g., a filter) passes through and emerges distal of an elongate channel, such as a working channel of an endoscope or a channel of a catheter. There, disposed distal of the elongate channel, the balloon or other medical device may be expanded (e.g., inflated in the case of a balloon) for some reason, such as to widen a passageway, secure a stent, collect emboli, etc. The balloon may then be partially or completely deflated. Subsequent to deflation, the practitioner may seek to withdraw the balloon or other medical device from the patient by pulling the balloon or other medical device into and through the elongate channel. However, in some circumstances, withdrawal of the balloon or other medical device is difficult as the balloon or other medical device folds, bunches, deforms, and/or catches in a manner that prevents the balloon or other medical device from transitioning to a sufficiently compact state. In some circumstances, the failure of the balloon or other medical device to transition to a compact state suitable for straightforward withdrawal is due, at least in part, to friction between adjacent portions of an inner surface of a balloon.

In circumstances where a balloon cannot be readily withdrawn into the channel of an elongate medical device (e.g., a working channel of an endoscope), the practitioner may need to withdraw the entire elongate medical device from the patient. Such withdrawal both wastes time and complicates the procedure, as reinsertion and repositioning of an endoscope or other elongate device may be difficult or time-consuming under many circumstances.

Some of the medical devices and/or medical assemblies disclosed herein comprise a lubricant (e.g., a lubricant layer) that is disposed on an inner surface of a balloon body. The lubricant may reduce friction between adjacent portions of the inner surface of the balloon body as the balloon body is withdrawn into an elongate channel. Such reduced friction may facilitate withdrawal of the balloon body into the elongate channel by allowing portions of the balloon body to slide past one other so that the balloon body may adopt a more compact configuration. Further, disposing a lubricant on an inner surface of a balloon body may expand the types of materials from which the balloon body may be manufactured. Stated differently, a lubricant may be disposed on an inner surface of a balloon body that is made of material that would have been unsuitable for use as a balloon body in the absence of the lubricant due to the tendency of the material to stick to itself (and thus impede withdrawal of the balloon into an elongate channel). In other words, the lubricant may allow the balloon body to transition to a compact state needed for withdrawal despite the tendency of the material from which the balloon body is made to stick to itself.

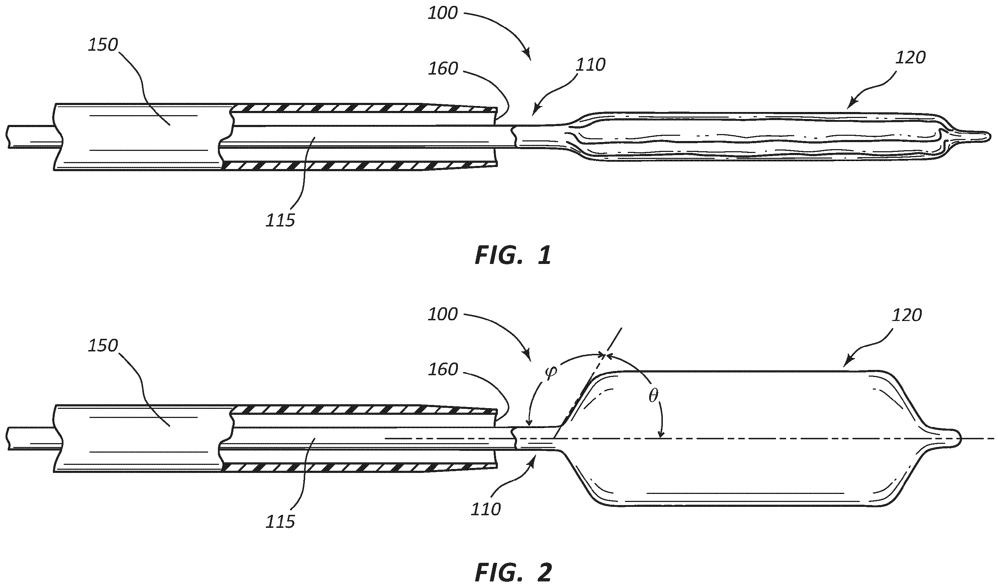

FIG. 1 provides a front view of a medical device assembly 100. The medical device assembly 100 comprises an elongate medical device 150 (such as a catheter or an endoscope) and a balloon catheter 110. The elongate medical device 150 comprises at least one elongate channel 160. In some embodiments, an elongate medical device may comprise other elongate channels as well. For example, in embodiments where the elongate medical device is an endoscope, the elongate medical device may comprise a working channel along with one or more other channels to deliver light and/or transmit an image to the viewer. The balloon catheter 110 may comprise a catheter 115 and a balloon 120 disposed generally distal of the catheter 115. Additionally, the balloon 120 may be coupled to and in fluid communication with the catheter 115.

When the components of the medical device assembly 100 are in the configuration depicted in FIG. 1, a portion of the catheter 115 is disposed within an elongate channel 160 of the elongate medical device 150, while the balloon 120 is disposed distal of the distal end of the elongate medical device 150. The balloon catheter 110 may be disposed in this manner by advancing the balloon catheter 110 through the elongate channel 160 until the balloon 120 is disposed distal of the elongate medical device 150. As the balloon catheter 110 is advanced through the elongate channel 160 of the elongate medical device 150, the balloon 120 may be in a relatively compact and uninflated state, such as that shown in FIG. 1. For example, the balloon may be folded on itself to adopt a compact configuration. In some instances, the balloon 120 may be packaged and supplied to the practitioner in this compact state. Such a relatively compact and uninflated state may allow insertion and advancement of the balloon catheter 110 through the channel 160 without generating substantial resistance, such as due to friction between the outer surface of the balloon 120 and the inner surface of the elongate medical device 150.

FIG. 2 provides a front view of the medical device assembly 100 in a configuration similar to that shown in FIG. 1, but with the balloon 120 in an inflated state. The medical device assembly 100 may be disposed in this manner by forcing fluid into the uninflated balloon 120 (see FIG. 1), thereby causing the balloon 120 to expand and adopt an inflated state (see FIG. 2). In some embodiments, the balloon 120 is inflated by forcing fluid, such as gas and/or liquid, into the balloon 120. More particularly, in some embodiments, the inflation fluid comprises water or an aqueous solution, such as a saline solution.

The balloon 120 may be expanded for any reason. For example, in some embodiments, the balloon 120 is inflated within a lumen of a patient to expand a stent that is disposed within the lumen. More particularly, in some embodiments, an esophageal stent may be deployed in the esophagus of a patient. The balloon 120 may be inserted into the interior of the stent such that inflation of balloon 120 causes the stent to expand radially away from the longitudinal axis of the stent. Such expansion may secure (or more fully secure) the stent within the esophagus. Stents placed at other locations within a patient may be secured in a similar manner.

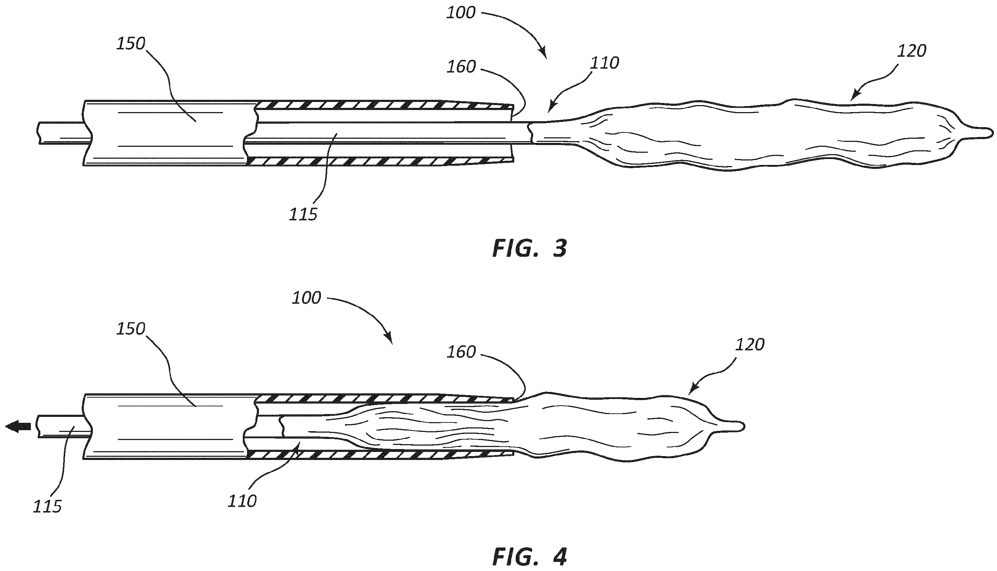

FIG. 3 provides a front view of the medical device assembly 100 in a configuration similar to that shown in FIGS. 1 and 2, except that the balloon 120 is in a deflated state that differs somewhat from the compact uninflated state (e.g., the state of the balloon when supplied from the manufacturer) depicted in FIG. 1. After the balloon 120 has been inflated as shown in FIG. 2, the balloon 120 may be deflated by removing a substantial portion of the fluid that had previously been forced into the balloon 120. When deflated in this manner, the balloon 120 may adopt an irregular configuration, such as that shown in FIG. 3, that differs from the compact state of the balloon 120 when it was advanced through the elongate medical device 150 (see FIG. 1). More particularly, in some embodiments, a balloon 120 in this deflated state may not be as compact as the balloon 120 was when it was advanced through the elongate medical device 150 (compare FIGS. 1 and 3). Stated otherwise, the deflated balloon 120 (see FIG. 3) may have a greater exposed surface area, occupy a larger volume, or define one or more dimensions that are of greater length than the balloon 120 did when it was initially advanced through the elongate medical device 150. Further, the balloon 120 may fold on itself, bunch, deform, or catch on itself in an irregular manner upon deflation.

FIG. 4 provides a front view of the medical assembly 100 in a configuration that differs from the configurations shown in FIGS. 1-3. This configuration shows the withdrawal of the balloon 120 into the channel 160 of the medical device 150 after the balloon 120 has been inflated (see FIG. 2) and deflated (see FIG. 3).

In some embodiments, withdrawal of the balloon 120 into the elongate medical device 150 after the balloon 120 has been inflated and deflated may require a proximal force that is greater than the proximal force that would have been required to withdraw the balloon 120 into the elongate medical device 150 if the balloon 120 were in the same configuration as it was when it was delivered through the elongate channel 160 (e.g., the configuration shown in FIG. 1).

The increase in force needed to withdraw the balloon 120 into the elongate medical device 150 may be caused, at least in part, by the balloon's 120 resistance to transitioning from a less compact configuration to a more compact configuration. Such resistance may be due to one or more factors. For example, upon deflation, the balloon 120 may collapse on itself such that the balloon 120 adopts a relatively non-compact configuration where portions of the inner surface of the balloon 120 contact one another. Such contact may create friction that makes the balloon 120 resistant to transitioning to a more compact configuration as the balloon 120 is withdrawn into the elongate medical device 150. Stated differently, friction arising from the contact of portions of the inner surface of the balloon 120 with each other may prevent portions of the balloon 120 from sliding past one another as needed for the balloon 120 to transition to a shape that is suitable for facile withdrawal of the balloon 120 into the channel 160 of the elongate medical device 150.

Friction between portions of the inner surface of a balloon 120 may be decreased by disposing a lubricant layer on the inner surface of the balloon 120. As the balloon 120 is withdrawn into the channel 160 of the elongate medical device 150, the lubricant layer may decrease the friction between portions of the inner surface of balloon 120, thereby allowing the balloon 120 to more readily transition from a less compact state to a more compact state. In other words, the lubricant layer may decrease the amount of force needed to withdraw the balloon 120 into the channel 160 of the elongate medical device 150 after the balloon 120 has been inflated and deflated relative to an identical balloon that lacks the lubricant layer.

Large balloons may generally be more difficult to withdraw into a particular working channel (e.g., a standard endoscope working channel) than small balloons. For example, a first balloon that has a first inflated diameter (and lacks a lubricant layer) may be more difficult to withdraw into the elongate channel than a second balloon (also lacking a lubricant layer) that has a second inflated diameter that is less than the first inflated diameter. For example, it may be more difficult to withdraw a balloon with an inflated diameter of 10 mm (and lacking a lubricant layer) into the channel of an elongate medical device than to withdraw a similar balloon with an inflated diameter of less than 10 mm into the same channel. Additionally, the difficulty of withdrawing a balloon into a channel may increase as the inflated diameter of the balloon increases. Thus balloons (lacking a lubricant layer) with an inflated diameter of greater than 12, 13, 14, 15, 16, 17 and/or 18 mm may be increasingly difficult to withdraw into an elongate channel than balloons that have smaller inflated diameters. Thus, disposition of a lubricant layer on an inner surface of a relatively large balloon may facilitate this withdrawal.

In addition to inflation diameter, other balloon characteristics may also affect the ability of a balloon to be withdrawn into a channel of an elongate medical device. For example, the shape of a balloon may affect the ability of a balloon to be withdrawn into an elongate channel. Some balloons may be shaped such that a line tangent with the outer surface of the balloon, when the balloon is inflated, intersects the longitudinal axis of the balloon body at a particular acute angle (θ) and a supplementary obtuse angle (φ) (see FIG. 2). Each balloon may have a plurality of tangent lines that intersect with the longitudinal axis of the balloon, each defining a particular acute angle θ. The maximum (i.e., largest) acute angle for each balloon may be referred to as "θ'". In some embodiments, θ' is greater than or equal to 60°, 70°, 75°, 80°, or 85°. The ease of withdrawing a balloon into a channel may be affected by the value of θ'. In other words, embodiments where θ' is relatively large may generally be more difficult to withdraw into an elongate channel than embodiments where θ' is relatively small. Thus, disposing a lubricant layer on the inner surface of balloon bodies where θ' is relatively large may allow these balloon bodies to be withdrawn into an elongate channel in spite of the difficulty of withdrawal that is typically associated with such balloon bodies.

The ease of withdrawing a balloon into a channel may be affected by the size of the channel. For example, balloons withdrawn through relatively small elongate channels may be more likely to become stuck. In some embodiments, balloons are withdrawn into a channel (such as a working channel of endoscope) that has a diameter of less than or equal to 3.8, 2.8, or 2.0 mm. In other or further embodiments, the ratio of the diameter of a balloon to the diameter of the channel through which it is to be withdrawn is greater than 2.5, 4.0, 5.0, and/or 6.0. To facilitate withdrawal of a balloon body into a relatively narrow channel, a lubricant layer may be disposed on an inner surface of the balloon body. Relatedly, disposition of a lubricant on an inner surface of a balloon may also allow for the deployment and withdrawal of a balloon through an elongate device with a relatively small channel. In other words, relative to balloons that lack an inner lubricant layer, balloons with an inner lubricant layer may be deployed through smaller elongate medical devices, which may cause less trauma to a patient upon insertion and withdrawal.

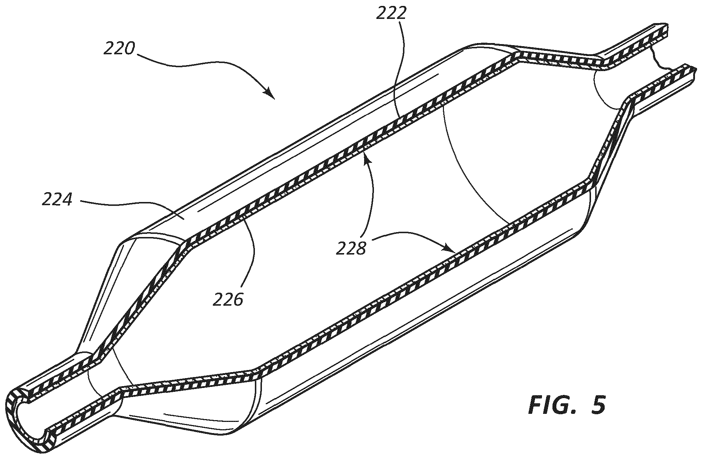

FIG. 5 provides a cut-away perspective view of a balloon 220 that resembles the balloon 120 described above in certain respects. Accordingly, like features are designated with like reference numerals, with the leading digits incremented to "2." Relevant disclosure set forth above regarding similarly identified features thus may not be repeated hereafter. Moreover, specific features of the balloons and other components shown in FIGS. 1-4 may not be shown or identified by a reference numeral in the drawings or specifically discussed in the written description that follows. However, such features may clearly be the same, or substantially the same, as features depicted in other embodiments and/or described with respect to such embodiments. Accordingly, the relevant descriptions of such features apply equally to the features of the balloon 220 depicted in FIG. 5. Any suitable combination of the features, and variations of the same, described with respect to the balloon 120 and related components illustrated in FIGS. 1-4, can be employed with the balloon 220 and related components of FIG. 5, and vice versa. This pattern of disclosure applies equally to further embodiments depicted in subsequent figures and described hereafter, wherein the leading digits may be further incremented.

FIG. 5 provides a cut-away perspective view of a balloon 220 that comprises a balloon body 222 (with an outer surface 224 and an inner surface 226), and a lubricant layer 228 disposed on the inner surface 226 of the balloon body 222.

The balloon body 222 may comprise PEBAX, Nylon, polyurethane, latex, or some other material. For example, a first material, such as PEBAX, may define the balloon body 222. The material from which the balloon body 222 is made may have a tendency to stick to itself. In other words, when a first surface of this material is in contact with a second surface of this material, the friction and/or adhesive forces between the contacting materials may prevent or otherwise impede the materials from sliding or slipping past each other. Thus, balloons that are made from such material and lack a lubricant layer may be resistant to changes in configuration that require balloon materials to slide past one another. For example, in instances where balloons have been deflated such that opposing sides of the balloon have come in contact with one another, friction between opposing sides may resist relative movement, causing the sides to "stick" to one another. Resistance to such changes in configuration may be one reason why balloons that lack a lubricant layer on the inner surface of the balloon body may require more force to transition to a compact state suitable for withdrawal through an elongate channel than balloons with a lubricant layer disposed on the inner surface of the balloon body.

A variety of lubricant layers 228 may be used to facilitate the withdrawal of a balloon 220 into a channel. For example, the lubricant layer 228 may be hydrophilic or hydrophobic. Such lubricant layers may comprise or consist essentially of one or more of the following: silicone oil, hyaluronic acid, Gantrez (i.e., copolymers of monoalkyl esters of poly(methyl vinyl ether/maleic acid)), polyvinylpyrrolidone (PVP), polyacrylamide (PA), PVP/PA copolymer, carboxyalkyl cellulose, hydroxyalkyl cellulose, alginate, and carbomers. For example, a hydrophobic lubricant layer (e.g., a layer comprising or consisting essentially of silicone oil) may be disposed on an inner surface 226 of the balloon body 222. In some embodiments, silicone oil may be sprayed onto an inner surface 226 of the balloon body 222. The silicone oil may remain on the inner surface of balloon body 226 after the balloon 220 has been inflated with water or an aqueous solution, as the silicone oil, due to its hydrophobic nature, does not dissolve to a significant extent in the water solution. This silicone oil layer may cause a reduction in friction between adjacent inner surfaces of the balloon body 226.

In some embodiments where the balloon 220 is inflated by forcing water or an aqueous solution into the balloon 220, an oil lubricant layer, such as silicone, on the inner surface 226 of the balloon body 222 may form droplets in the water, depending on the lubricant and the inflation fluid. In some instances, the presence of droplets may distort or impair the field of view seen through an endoscope, where the object being viewed is on the other side of the balloon 220 (e.g., due to light refraction across the droplets).

In some embodiments, the oil lubricant and the inflation fluid may be selected to minimize or reduce the amount of refraction that occurs as objects are viewed through the endoscope. For example, the refractive index of the inflation fluid and/or oil may be matched to one other (or to the refractive index of the balloon body), thereby minimizing the amount of distortion that occurs as objects are viewed through the balloon 220.

In some embodiments, a hydrophilic coating may be deposited on the inner surface 226 of the balloon body 222, thereby functioning as a lubricant layer. A balloon 220 where the balloon body 222 has been coated with a hydrophilic layer in this manner may be inflated by forcing an aqueous solution or mixture into the balloon 220 and subsequently deflated by removing most of the solution or mixture from the balloon 220. The lubricant layer 228 may retain some water that adsorbs to the hydrophilic surface. The retention of water on the inner surface of the hydrophilic coating may decrease the friction between adjacent portions of the hydrophilic coating. In other words, the combination of the hydrophilic coating and the water retained thereon may decrease the friction between portions of the balloon 220 that are brought adjacent to each other as the balloon 220 is deflated.

In some embodiments, the use of a hydrophilic coating may reduce or eliminate droplet formation, as the hydrophilic coating may be permanently bonded to the inner diameter of the balloon 220.

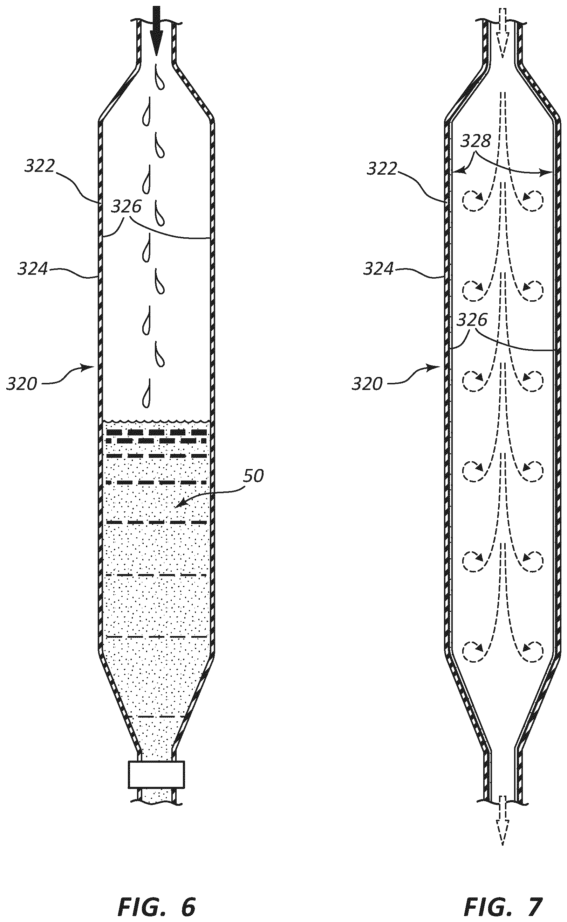

FIG. 6 provides a cross-sectional front view of a balloon 320 that is being filled with a solution 50 for providing a lubricant layer on the inner surface 326 of the balloon body 322. A balloon 320 with an inner lubricant layer may be manufactured in any suitable manner. In one embodiment, a method of manufacturing a balloon 320 with an inner lubricant layer may comprise obtaining a balloon body 322, applying a coating on an inner surface 326 of the balloon body 322, and curing the coating such that the cured coating provides a bonded, fixed, and/or permanent lubricant layer on the inner surface of the balloon body 322.

In one embodiment, as shown in FIG. 6, a balloon body 322 may be partially or completely filled with a solution 50 comprising a polymer (e.g., PVP), water, an alcohol, and a cross-linking catalyst. After the balloon body has been partially or completely filled, the solution 50 may be drained from the balloon body 322 at a controlled rate (e.g., 6.25-12.5 mL/minute). Liquid retained on the inner surface 326 of the balloon body 322 may be dried and/or cured in any suitable manner to create a bonded, fixed, stable, and/or solid inner lubricant layer (e.g., a solid hydrophilic PVP layer). In some embodiments, the drying and/or curing process may comprise one or more of forcing air into the balloon 320, exposing the balloon 320 to UV light, exposing the balloon 320 to heat, and chemically curing the lubricious coating.

More particularly, in some embodiments where UV light is used to partially or completely cure the layer, the balloon 320 may be exposed to UV light by disposing a UV light source outside of the balloon 320. The UV light emitted by the UV light source may pass through the balloon body 322 and promote cross-linking of a lubricant layer (e.g., a PVP layer) disposed on the inner surface 326 of the balloon body 322.

FIG. 7 depicts air being pumped through the balloon of FIG. 6 where most of the solution 50 has been drained from the balloon body 322. As noted above, forcing air through the balloon in this manner may help dry and/or cure the lubricant layer 328.

Lubricant layers may also be used on the interior of devices other than balloons to facilitate their withdrawal into an elongate channel of an elongate medical device. For example, a lubricant layer may be applied to the interior of a filter or basket that has been inserted through an elongate channel. In a manner analogous to that described above in connection with a balloon, the lubricant layer may facilitate compaction of the filter as the filter is withdrawn into the channel. For example, as the filter is withdrawn into an elongate channel of an elongate medical device, the inner lubricant layer may allow interior surfaces of the filter to slide past one another to facilitate compaction and withdrawal of the filter.

Lubricant layers may also be disposed on the inner surface of angioplasty balloons, valvuloplasty balloons, or any other balloon (e.g., balloons used to stabilize passageways for instruments within a patient).

### Example 1-Coating of a balloon with a hydrophilic layer

Balloon bodies of various sizes were filled with an water/isopropyl alcohol solution containing polyvinylpyrrolidone and cross-linking catalyst. After filling each balloon, the solution was drained at a controlled rate between 6.25 and 12.5 mL/minute. After being drained, a layer of solution remained on the inner surface of the balloon, forming a wet interior surface. The balloon body was dried by forcing air through the balloon body and then exposed to UV light (with the lamp outside of the balloon). The resultant balloon had an exterior layer formed from material of the balloon body and a hydrophilic interior layer that included polyvinylpyrrolidone. It was observed that the hydrophilic coating did not substantially diminish the ability of a practitioner to look through the clear balloon to observe objects on the other side of the balloon.

### Example 2

To investigate whether and to what extent a lubricant on the inner surface of a balloon might have on the force needed to withdraw a balloon into a channel after the balloon had been inflated and deflated, three sets of eight 18-19-20 mm × 8 cm multi-stage balloons were obtained. The inner surfaces of the balloons in the first set were coated with a hydrophilic (i.e., PVP) coating as described in Example 1. The second set of balloons was not coated with a lubricant layer. The inner surfaces of the third set of balloons were sprayed with a silicone oil lubricant prior to inflation to form a lubricant layer of silicone oil.

Each of the 24 balloons was advanced within a 2.8 mm diameter working channel of an endoscope such that each balloon was disposed distal of the distal end of the working channel. The balloons were then inflated to the maximum rated pressure for 30 seconds and then deflated by vacuum. With the balloon catheter still under vacuum and the working channel in a straight orientation, a proximal force was applied to pull the balloon into the working channel of the endoscope at a rate of 500 mm per minute. The magnitude of the force was monitored, and the maximum force needed to pull the balloon into the working channel of the endoscope (i.e., the maximum withdraw load) was recorded. The average maximum withdraw load for each set of balloon catheters was also calculated.

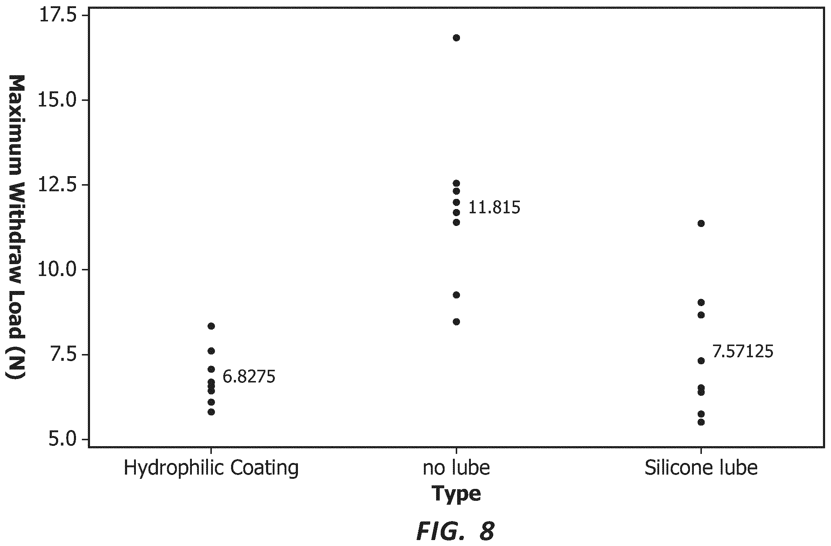

The results are shown in FIG. 8, which is a column scatter graph depicting the maximum force needed to withdraw the balloons into the working channel of the endoscope. The graph shows that the force needed to withdraw a balloon with no lubricant layer on the inner surface (see the center column of graph) is generally higher than the force needed to withdraw a balloon which had either a silicone lube inner layer or a hydrophilic coating disposed on the inner surface.

The average force needed to withdraw balloons that had been coated with a hydrophilic layer into the channel of the working endoscope was 6.8 Newtons (N). The average force needed to withdraw balloons that had been lubricated by spraying silicone on an inner surface was 7.6 N. The average force needed to withdraw multi-stage balloons that lacked an inner lubricant layer into the channel of the endoscope was 11.8 N.

### Example 3

To investigate the degree to which various coatings decrease friction between adjacent inner surfaces of a balloon body, the "test peak friction force" for various balloon materials was measured as described below.

First, three sets of 7033 SA01 PEBAX balloon bodies were obtained. The inner surfaces of the balloon bodies in the first set were coated with the hydrophilic PVP coating as described above in Example 1. The second set of balloons was not coated with a lubricant layer. The third set of balloons was coated with a hydrophobic layer by spraying silicone oil with a nominal viscosity of 100 mPa·S on the inner surface of the balloons.

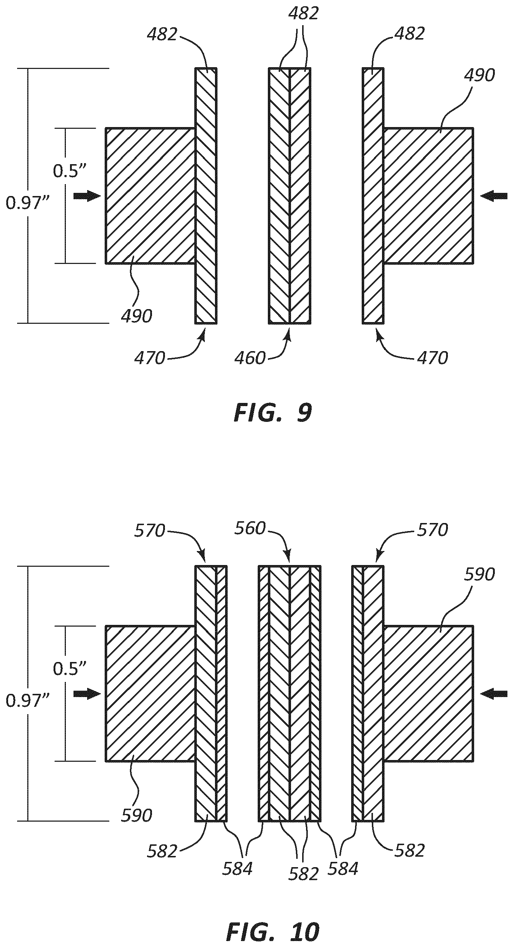

Each of the balloons was then inverted (thereby exposing the inner surface of the balloon), cut into lengths of approximately 0.97 inches, and flattened. FIG. 9 depicts the setup used to measure the test peak friction force of a balloon segment 460 that lacks a lubricant layer (e.g., a balloon segment of a balloon defined by a first material 482). FIG. 10 depicts the setup for measuring the test peak friction force of balloon segments 560 with a first material 582 and a second material 584 disposed on the inner surface of the first material 582.

As depicted in FIG. 9, to ascertain the test peak friction force for a balloon segment 460 that lacks a lubricant layer, a flattened balloon segment 460 was sandwiched between a sheet 470 of balloon material 482 that was identical with the material of the balloon segment 460 while the entire testing system was submerged in a saline bath (37 °C). The sheets 470 of balloon material 482 were attached to two 80A durometer silicone pads 490. With these components disposed in this manner, the sandwich structure was compressed by moving the silicone pads 490 toward each other to provide a normal force (100 gram-force; 0.98 × 10⁻³ N) to the inverted balloon segment 460. The inverted balloon segment 460 was then pulled upward at a rate of 1 cm/s for 1 cm, and the maximum force required to maintain this pull rate (i.e., the test peak friction force) was recorded by a force gauge coupled to the inverted balloon segment 460.

The test peak friction forces for balloon segments 560 that had been coated with either silicone oil or PVP were measured according to an analogous procedure. As depicted in FIG. 10, the inverted balloon segment 560 (comprising a first material 582 and a second material 584 (silicone oil or PVP) disposed on the inner surface of the first material 582) was disposed between two sheets 570 that each comprise a first material 582 and a second material 584 disposed on the inner surface of the first material 582. For each sheet 570, the second material 584 is oriented toward the inverted balloon segment 560. The test peak friction force was then measured as described above in connection with the balloon segment 460 that lacks a lubricant layer by compressing the sandwich structure and pulling the inverted balloon segment 560 in an upward direction at a constant rate.

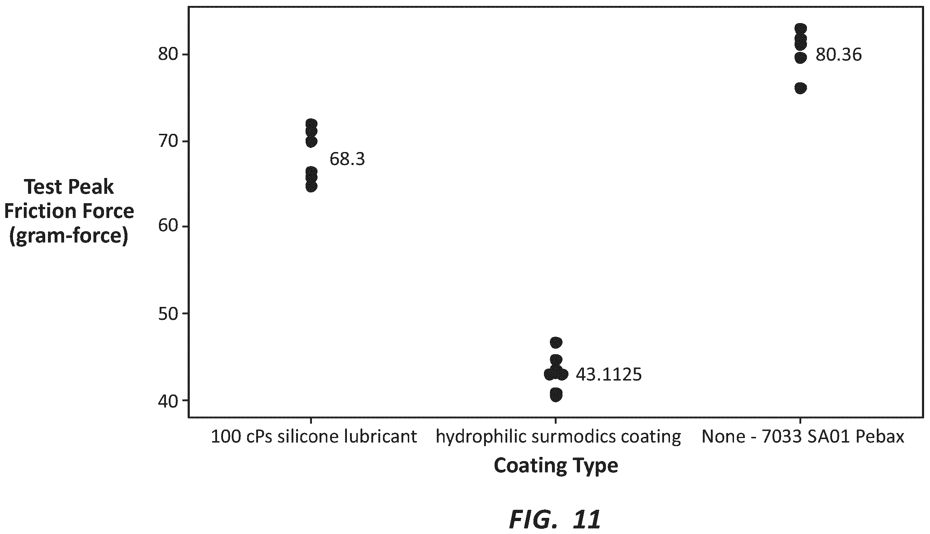

The results of these experiments are shown in FIG. 11, which is a column scatter graph depicting the test peak friction force for each balloon segment. On average, the test peak friction force for balloon segments that had been coated with a hydrophobic (i.e., silicone oil) or hydrophilic (i.e., PVP) layer was lower than the test peak friction force for balloon segments that had lacked a lubricant layer. More particularly, the average test peak friction force for balloon segments that had been lubricated with silicone oil spray was 68.3 gram-force, a 15% reduction in test peak friction force relative to the average test peak friction force for a balloon segment that had lacked a lubricant layer (80.4 gram-force). The average test peak friction force for balloon segments that had been coated with a hydrophilic PVP layer was 43.1 gram-force, a 46% reduction in test peak friction force relative to the average test peak friction force for a balloon segment that had lacked a lubricant layer (80.4 gram-force). In other words, the test peak friction force for balloon segments having a second material disposed on the inner surface of a first material may be at least 10%, 15%, 25%, 35%, or 45% lower than the test peak friction force for balloon segments of only first material. Further, the data shown in FIG. 11 demonstrate that the test peak friction force for a balloon segment comprising a first material with a second material disposed on the inner surface of the first material may be less than 75, 70, 65, 60, 55, 50, and/or 45 gram-force.

Any methods disclosed herein include one or more steps or actions for performing the described method. The method steps and/or actions may be interchanged with one another. In other words, unless a specific order of steps or actions is required for proper operation of the embodiment, the order and/or use of specific steps and/or actions may be modified. Moreover, sub-routines or only a portion of a method described herein may be a separate method within the scope of this disclosure. Stated otherwise, some methods may include only a portion of the steps described in a more detailed method.

Reference throughout this specification to "an embodiment" or "the embodiment" means that a particular feature, structure or characteristic described in connection with that embodiment is included in at least one embodiment. Thus, the quoted phrases, or variations thereof, as recited throughout this specification are not necessarily all referring to the same embodiment.

Similarly, it should be appreciated by one of skill in the art with the benefit of this disclosure that in the above description of embodiments, various features are sometimes grouped together in a single embodiment, figure, or description thereof for the purpose of streamlining the disclosure. This method of disclosure, however, is not to be interpreted as reflecting an intention that any statement require more features than those expressly recited in that statement. Rather, as the following statements reflect, inventive aspects lie in a combination of fewer than all features of any single foregoing disclosed embodiment. Thus, the statements following this Detailed Description are hereby expressly incorporated into this Detailed Description, with each statement standing on its own as a separate embodiment. This disclosure includes all permutations of the independent statements with their dependent statements.

Recitation in the statements of the term "first" with respect to a feature or element does not necessarily imply the existence of a second or additional such feature or element. It will be apparent to those having skill in the art that changes may be made to the details of the above-described embodiments without departing from the underlying principles of the present disclosure.

### STATEMENTS OF INVENTION

1. A balloon for use in medical procedures, the balloon comprising:
   a first material defining a balloon body; and
   a second material disposed on an inner surface of the balloon body;
   wherein a test peak friction force for a balloon segment comprising the first material with the second material disposed on the inner surface of the first material is less than a test peak friction force for a balloon segment of only the first material.
2. The balloon of statement 1, wherein the test peak friction force for the balloon segment comprising the first material with the second material disposed on the inner surface of the first material is at least 10% lower than the test peak friction force for the balloon segment of only the first material.
3. The balloon of any one of statements 1-2, wherein the test peak friction force for the balloon segment comprising the first material with the second material disposed on the inner surface of the first material is at least 25% lower than the test peak friction force for the balloon segment of only the first material.
4. The balloon of any one of statements 1-3, wherein the test peak friction force for the balloon segment comprising the first material with the second material disposed on the inner surface of the first material is at least 45% lower than the test peak friction force for the balloon segment of only the first material.
5. The balloon of any one of statements 1-4, wherein the test peak friction force for the balloon segment comprising the first material with the second material disposed on the inner surface of the first material is less than 75 gram-force.
6. The balloon of any one of statements 1-5, wherein the test peak friction force of a sheet of first material with second material disposed on the inner surface of the first material is less than 60 gram-force.
7. The balloon of any one of statements 1-6, wherein the test peak friction force of a sheet of first material with second material disposed on the inner surface of the first material is less than 50 gram-force.
8. A balloon for use in medical procedures, the balloon comprising:
   a balloon body configured for delivery through a channel of an elongate medical device; and
   a lubricant layer disposed on an inner surface of the balloon body;
   wherein the lubricant layer is configured to decrease the amount of force needed to withdraw the balloon body into the channel of the elongate medical device after the balloon body has been inflated and deflated relative to an identical balloon that lacks the lubricant layer.
9. The balloon of statement 8, wherein the lubricant layer is configured to facilitate compaction of the balloon body as the balloon body is withdrawn into the channel of the elongate medical device.
10. The balloon of any one of statements 8-9, wherein the lubricant layer comprises a liquid.
11. The balloon of any one of statements 8-10, wherein the lubricant layer comprises a polymer.
12. The balloon of any one of statements 8-11, wherein the lubricant layer comprises silicone.
13. The balloon of any one of statements 8-9 and 11, wherein the lubricant layer comprises a solid hydrophilic layer.
14. The balloon of statement 13, wherein the lubricant layer comprises polyvinylpyrrolidone.
15. The balloon of any one of statements 8-14, wherein the balloon body, when inflated, comprises an exterior surface such that a line tangent with the exterior surface intersects a longitudinal axis of the balloon body at an acute angle that is greater than 65 degrees.
16. The balloon of statement 15, wherein the acute angle is greater than 80 degrees.
17. The balloon of any one of statements 8-16, wherein the balloon is configured to expand an esophageal stent.
18. The balloon of any one of statements 8-17, wherein the balloon body comprises PEBAX.
19. The balloon of any one of statements 8-17, wherein the balloon body comprises Nylon.
20. The balloon of any one of statements 8, 9, 11, and 13-19, wherein the lubricant comprises a layer of polyvinylpyrrolidone that has been cured by a process comprising exposure to UV light.
21. A medical device assembly comprising:
   a balloon comprising a balloon body and a lubricant layer disposed on an inner surface of the balloon body; and
   an elongate medical device comprising an elongate channel;
   wherein the balloon body is configured for delivery through the elongate channel of the elongate medical device, and the lubricant layer decreases the amount of force needed to withdraw the balloon body into the elongate channel of the elongate medical device after the balloon body has been both inflated and deflated relative to an identical balloon that lacks the lubricant layer.
22. The assembly of statement 21, wherein the balloon comprises a diameter of greater than 12 mm when inflated and the elongate channel of the elongate medical device comprises a diameter of 3.8 mm or less.
23. The assembly of any one of statements 21-22, wherein the balloon comprises a diameter of greater than or equal to 14 mm when inflated and the elongate channel of the elongate medical device comprises a diameter of 2.8 mm or less.
24. The assembly of any one of statements 21-23, wherein the balloon comprises a diameter of greater than or equal to 18 mm when inflated and the elongate channel of the elongate medical device comprises a diameter of 2.8 mm or less.
25. The assembly of any one of statements 21-24, wherein the ratio of the diameter of the balloon when inflated to the diameter of the elongate channel is greater than 4.
26. A method of manufacturing a balloon for deployment within a cavity of patient, the method comprising:
   obtaining a balloon body;
   applying a coating on an inner surface of the balloon body; and
   curing the coating;
   wherein the cured coating provides a permanent lubricant on the inner surface of the balloon body.
27. The method of statement 26, wherein applying the coating comprises at least partially filling the balloon body with a mixture comprising a polymer and a catalyst.
28. The method of statement 27, wherein the polymer comprises polyvinylpyrrolidone.
29. The method of any one of statements 26-28, wherein curing the coating comprises forcing air into the interior of the balloon body.
30. The method of any one of statements 26-29, wherein curing the coating comprises exposing the coating to UV light.
31. A method of using a balloon during a medical procedure, the method comprising:
   disposing a balloon in an uninflated state through an elongate channel of an elongate medical device such that the balloon emerges from a distal end of the elongate medical device;
   inflating the balloon distal of the distal end of the elongate medical device;
   deflating the balloon while the balloon is distal of the distal end of the elongate medical device; and
   withdrawing the balloon into the elongate channel of the elongate medical device;
   wherein the balloon comprises an inner lubricant layer for facilitating withdrawal of the balloon into the elongate channel of the elongate medical device.

## Claims

1. A balloon (220) for use in medical procedures, the balloon comprising:
a balloon body (222) configured for delivery through a channel of an elongate medical device (150); and
a lubricant layer (228) disposed on an inner surface (226) of the balloon body (220);
wherein the lubricant layer (228) is configured to decrease the amount of force needed to withdraw the balloon body (222) into the channel of the elongate medical device (150) after the balloon body (222) has been inflated and deflated relative to an identical balloon that lacks the lubricant layer.

2. The balloon (220) of claim 1, wherein the lubricant layer is configured to facilitate compaction of the balloon body (222) as the balloon body (222) is withdrawn into the channel of the elongate medical device (150).

3. The balloon (220) of claim 1 or 2, wherein the lubricant layer (228) comprises a liquid.

4. The balloon (220) of any one of claims 1-3, wherein the lubricant layer (228) comprises a polymer.

5. The balloon (220) of any one of claims 1-4, wherein the lubricant layer (228) comprises silicone.

6. The balloon (220) of any one of claims 1-2 and 4, wherein the lubricant layer (228) comprises a solid hydrophilic layer.

7. The balloon (220) of claim 6, wherein the lubricant layer (228) comprises polyvinylpyrrolidone.

8. The balloon (220) of any one of claims 1-7, wherein the balloon body (222), when inflated, comprises an exterior surface such that a line tangent with the exterior surface intersects a longitudinal axis of the balloon body at an acute angle that is greater than 65 degrees.

9. The balloon (220) of claim 8, wherein the acute angle is greater than 80 degrees.

10. The balloon (220) of any one of claims 1-9, wherein the balloon is configured to expand an esophageal stent.

11. The balloon (220) of any one of claims 1-10, wherein the balloon body (222) comprises PEBAX.

12. The balloon (220) of any one of claims 1-10, wherein the balloon body (222) comprises Nylon.

13. The balloon (220) of any one of claims 1, 2, 4, and 6-12, wherein the lubricant comprises a layer of polyvinylpyrrolidone that has been cured by a process comprising exposure to UV light.

14. A medical device assembly comprising (100):
the balloon (220) of any one of claims 1-13; and
an elongate medical device (150) comprising an elongate channel (160),
wherein the balloon body (222) is configured for delivery through the elongate channel (160) of the elongate medical device (150).

15. The assembly (100) of claim 14, wherein the balloon (220) comprises a diameter of greater than 12 mm when inflated and the elongate channel (160) of the elongate medical device (150) comprises a diameter of 3.8 mm or less.
